# EUROPEAN PATENT SPECIFICATION

(11) **EP 3 940 534 B1**
(45) Date of publication and mention of the grant of the patent: **24.07.2024**
(21) Application number: 20810050.3
(22) Date of filing: 23.03.2020
(51) Int. Cl.: H04L 9/40, G06F 9/4401, A24F 40/50, A24F 40/65, A61M 11/04, A61M 15/00, A61M 15/06, A61M 16/00, G06F 21/62, G16H 40/63, G16H 20/13, G16H 40/67

(54) **METHOD OF CONTROLLING VAPORIZING DEVICE, VAPORIZING DEVICE, AND CONTROL SYSTEM**
VERFAHREN ZUR STEUERUNG EINER ZERSTÄUBUNGSVORRICHTUNG SOWIE ZERSTÄUBUNGSVORRICHTUNG UND STEUERSYSTEM
PROCÉDÉ DE COMMANDE D'APPAREIL D'ATOMISATION ET APPAREIL D'ATOMISATION, ET SYSTÈME DE COMMANDE

(30) Priority: 21.05.2019 CN 201910431791
(43) Date of publication of application: 19.01.2022
(73) Proprietor: Huizhou Happy Vaping Technology Limited, Huizhou, Guangdong 516000 (CN)
(72) Inventor: LIN, Guangrong, Shenzhen, Guangdong 518104 (CN); ZHENG, Xianbin, Shenzhen, Guangdong 518104 (CN); ZHANG, Xiyong, Shenzhen, Guangdong 518104 (CN)
(74) Representative: Meyer, Thorsten
(86) International application number: PCT/CN2020/080630
(87) International publication number: WO 2020/233216

(56) References cited:
- CN-A- 108 853 661
- CN-A- 108 853 661
- CN-A- 109 498 921
- CN-A- 109 581 937
- CN-A- 110 209 441
- US-A1- 2013 228 169
- US-A1- 2013 340 775
- US-A1- 2016 325 055
- US-A1- 2019 014 824

## Description

### TECHNICAL FIELD

The disclosure relates to the field of vaporizing devices, more particularly to a method of controlling a vaporizing device, a vaporizing device, and a control system.

### BACKGROUND

The vaporizing device usually heats and vaporizes liquid substances or paste-like substances, such as drugs and E-cigarette liquid, to generate aerosol or vapor for users to use. As people pay more attention to their health, they realize that overuse may be harmful to health.

According to existing methods of controlling a vaporizing device, the users usually can freely use the device without any appropriate restriction or control during using. In particular, the amount of the intake of vaporized aerosol or vapor cannot be calculated, controlled or restricted until the substances to be vaporized, such as drugs and E-cigarette liquid, run out. In such a case, the users may overuse after a long period of use and the excessive intake probably will be harmful to health.

In particular, if users need to use a medicine with the intake or administration dosage controlled by medical personnel, it is necessary for the vaporizing device to be operated under a remote control of a regulator or authorized medical personnel, and it is desired to adjust the limit value of administration dosage in real-time based on user's personal information including physical condition and use condition.

Reference document US 2019014824 A1 discloses a communication system comprising: a vaporizer device 100 for vaporizing materials, a computing device 401, a database 502, a processing unit 503, and a communication network 504. A healthcare provider could use the processing unit 503 to monitor a patient's use of vaporizing materials by comparing the patient's actual dosage against a prescribed dosage. If the healthcare provider suspects the patient may be abusing vaporizing materials, the healthcare provider can use the processing unit 503 to lock the vaporizing device, and thereby prevent any further abuse.

Reference document US 2016/325055 A1 discloses an apparatus for delivering cannabidiol and associated compounds to promote health for patient for medical treatment. The apparatus has a vaporizer component comprising a heating element for vaporizing mixed compound. The reference document further teaches reducing the vaporization ratio when a cumulative dose is approaching the target dose.

Reference document US 2013/340775 A1 discloses a communication system for an electronic cigarette. The document further teaches disabling or reducing the power supplied to the heating element of an electronic cigarette if an internal counter indicates that the user's smoke rate is higher than is allowed or when an allowed number of puffs has been reached, or when the number of puffs that indicates a spent cartomizer is reached.

### SUMMARY

### Technical problem

An object of the disclosure is to overcome the above shortcomings and provide a method of controlling a vaporizing device, a vaporizing device, and a control system. The controlling method can adjust the limit value of administration dosage of the vaporizing device based on user's physical condition and use condition, and the vaporizing device and the control system are designed for implementing the control method.

### Technical solutions

The disclosure provides a technical solution as follow. A method of controlling a vaporizing device comprises: by a communication between an administrator terminal device and a cloud server, a user terminal device and a vaporizing device, obtaining various parameters of the vaporizing device by means of the administrator terminal device, writing a new control parameter into the vaporizing device by means of the administrator terminal device, and, when the new control parameter is triggered, automatically entering a new state of the vaporizing device to achieve a control for the vaporizing device;
the method further comprises operating steps of:
   (1) opening software of the administrator terminal device, to implement secure logon of an administrator;
   (2) inputting an identification code for the vaporizing device, to enter a parameter setting interface for the vaporizing device;
   (3) reading and analyzing relevant information of the vaporizing device;
   (4) writing a new control parameter based on a service condition of the vaporizing device and user's physical condition;
   (5) storing the new control parameter in the cloud server when the administrator terminal device is in communication with the cloud server;
   (6) storing the new control parameter in the user terminal device when the cloud server is in communication with the user terminal device;
   (7) writing the new control parameter into the vaporizing device when the user terminal device is in communication with the vaporizing device; wherein the new control parameter comprises a new limit value of aerosol intake; and
   (8) automatically entering a new state of the vaporizing device when the new control parameter is triggered; where the automatically entering a new state of the vaporizing device when the new control parameter is triggered comprises: when a value of aerosol intake detected by means of the vaporizing device exceeds the new limit value of aerosol intake, shutting down power output by means of a power control unit of the vaporizing device for a period of set OFF time;
the value of aerosol intake is detected by an air flow calculating unit, the power control unit, an energy statistics unit, and an energy and vaporizing amount conversion unit provided in the vaporizing device; where the air flow calculating unit is configured to calculate a value of air flow based on a modeling volume of a detecting air passage (5) and a change of suction pressure, the power control unit is configured to control output power that is output to a heating resistor (3) based on the value of air flow, the energy statistics unit is configured to calculate energy output to the heating resistor (3) based on the output power over a period of time, the energy and vaporizing amount conversion unit is configured to calculate the value of aerosol intake from the energy.

Preferably, the communication between an administrator terminal device and a cloud server, a user terminal device and a vaporizing device is implemented in such a manner that the administrator terminal device is in communication with the cloud server, the cloud server is in communication with the user terminal device, and the user terminal device is in communication with the vaporizing device.

Preferably, the method comprises the administrator terminal device and the vaporizing device being in direct communication with each other in a wired or wireless manner.

Preferably, the vaporizing device is configured with a first wired communication module or a first wireless communication module, wherein the first wired communication module or the first wireless communication module is in communication with a second wired communication module or a second wireless communication module of the user terminal device, to allow the user terminal device to read information of the vaporizing device and control the vaporizing device.

Preferably, the vaporizing device is provided with the identification code, by which the user terminal device is capable of creating a connection and communication with the vaporizing device.

Preferably, the cloud server is configured to store information or a program for identifying the identification code. Once the identification code is input, the administrator terminal device will be authorized to create a communication to the vaporizing device, to obtain various parameters and write the control parameter.

Preferably, during the step of automatically entering a new state of the vaporizing device when the new control parameter is triggered, further issuing an alarm by means of the administrator terminal device, or the user terminal device, or the vaporizing device.

Preferably, the limit value of aerosol intake comprises a limit value of single puff intake. That is, when a value of single puff intake exceeds the limit value of single puff intake, shutting down output by means of the power control unit of the vaporizing device for a period of the set OFF time.

Preferably, the limit value of aerosol intake comprises a limit value of total intake. That is, when a sum value of aerosol intake per unit time exceeds the limit value of total intake, shutting down output by means of the power control unit of the vaporizing device for a period of the set OFF time.

Preferably, the steps (4) to (8) specifically comprise operating steps of:
(1) Writing a new limit value of single puff intake, a new limit value of total intake and an OFF time based on a service condition of the vaporizing device and user's personal information;
(2) Storing the new limit value of single puff intake, the new limit value of total intake and the OFF time in the cloud server when the administrator terminal device is in communication with the cloud server;
(3) Storing the new limit value of single puff intake, the new limit value of total intake and the OFF time in the user terminal device when the cloud server is in communication with the user terminal device;
(4) Writing the new limit value of single puff intake, the new limit value of total intake and the OFF time into the vaporizing device when the user terminal device is in communication with the vaporizing device;
(5) Implementing controlling of the vaporizing device based on the new limit value of single puff intake, the new limit value of total intake and the OFF time, and, when a value of single puff intake exceeds the limit value of single puff intake or a sum value of aerosol intake per unit time exceeds the limit value of total intake, shutting down or reducing output by means of the power control unit of the vaporizing device;
(6) Returning to a standby state of the vaporizing device after a period of the set OFF time passes since output is shut down by means of the power control unit of the vaporizing device.

The disclosure provides another technical solution as follow. A vaporizing device comprises a battery, a control circuit board, a heating resistor, a vaporizing passage, a mouth piece, a detecting air passage, and an air pressure sensor. The air pressure sensor is disposed in the detecting air passage to detect the suction pressure. The control circuit board is further arranged with the microcontroller MCU, an air flow calculating unit, an energy statistics unit, an energy and vaporizing amount conversion unit, and a power control unit, which are configured to execute instructions during operation to implement the above controlling method. The air flow calculating unit is configured to calculate the corresponding value of air flow based on the modeling volume of the detecting air passage and the change of suction pressure. The power control unit is configured to control output power that is output to the heating resistor based on the value of air flow. The energy statistics unit is configured to calculate the energy output to the heating resistor, based on the output power and the period of time. The energy and vaporizing amount conversion unit is configured to calculate the corresponding value of aerosol intake from the energy. The microcontroller is configured to compare the corresponding value of aerosol intake with the preset limit value of aerosol intake and then send corresponding control signal to the power control unit. The power control unit is capable of shutting down for a certain period of time or reducing power output, to limit the aerosol intake.

The disclosure provides another technical solution as follow. A control system for the vaporizing device comprises the vaporizing device, the user terminal device, the cloud server, and the administrator terminal device, which are in communication with each other. The administrator terminal device is configured to obtain various parameters of the vaporizing device during communication, and write the new control parameter into the vaporizing device, and, the vaporizing device is configured to automatically enter a new state to achieve a control for the vaporizing device when the new control parameter is triggered.

Preferably, the user terminal device is disposed in the vaporizing device.

### Advantages

The method of controlling a vaporizing device according to the disclosure allows the authorized administrator to login and query the service condition of the vaporizing device by means of the administrator terminal device and to write new control parameters when necessary. The method provides the vaporizing device with a targeted remote control in particular on the limit value of aerosol or vapor intake, prevents the user from overusing the aerosol or vapor in single puff or in a unit time, and avoids health hazards. According to the controlling method, the vaporizing device is operated in a condition that it is loaded with control programs. Thus, it is not necessary to remotely control the device on line all the time, and the vaporizing device can be normally operated even without network communication.

### BRIEF DESCRIPTION OF THE DRAWINGS

FIG.1 is a diagram illustrating communication relation according to a controlling method of the disclosure;
FIG.2 is a flow chart of a controlling method according to the disclosure;
FIG.3 is another flow chart of a controlling method according to the disclosure;
FIG.4 is a cross-sectional view of a vaporizing device according to the disclosure;
FIG.5 is a functional block diagram of a circuit board of a vaporizing device according to the disclosure;

### DETAILED DESCRIPTION OF ILLUSTRATED EMBODIMENTS

In order to make purposes, technical solutions and advantages of the disclosure clearer, the disclosure will be further explained in detail with reference to drawings and embodiments described hereinafter. It should be understood that the specific embodiments described herein are only used to explain the present invention and are not intended to limit the present invention.

### Embodiments

The controlling method of the disclosure provides a remote use and control method which is targeted on the basis that the vaporizing device itself is capable of controlling the user's usage.

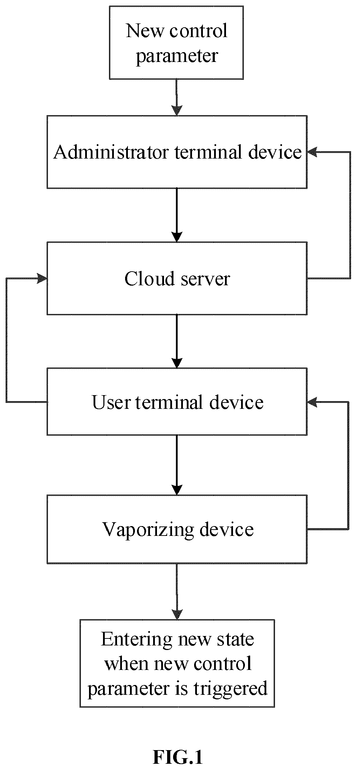

Referring to FIG. 1, a method of controlling a vaporizing device according to the disclosure comprises: by a communication between an administrator terminal device and a cloud server, a user terminal device and a vaporizing device, obtaining various parameters of the vaporizing device and writing a new control parameter into the vaporizing device by means of the administrator terminal device, and, when the new control parameter is triggered, automatically entering a state of the vaporizing device, thereby achieving controlling of the vaporizing device. In an embodiment, the communication between an administrator terminal device and a cloud server, a user terminal device and a vaporizing device is implemented in such a manner that the administrator terminal device is in communication with the cloud server, the cloud server is in communication with the user terminal device, and the user terminal device is in communication with the vaporizing device.

The new control parameter includes a limit value of aerosol intake. In such a case, when a value of aerosol intake detected by means of the vaporizing device exceeds the limit value of aerosol intake, the power control unit of the vaporizing device shuts down power delivery, i.e., the power is cut off and the vaporizing device is stopped. The limit value of aerosol intake includes a limit value of single puff intake and a limit value of total intake. The vaporizing device according to the method of the disclosure are loaded with relevant control programs, and is configured to control, once a new control parameter is written, the user's usage based on the new control parameter.

It is apparent that, the administrator terminal device, the cloud server, the user terminal device, and the vaporizing device according to the method of the disclosure are preinstalled with relevant application and control programs, and have communication function.

Herein, the vaporizing device is configured with a first wired communication module or a first wireless communication module, wherein the first wired communication module or the first wireless communication module is in communication with a second wired communication module or a second wireless communication module of the user terminal device, to allow the user terminal device to read information of the vaporizing device and control the vaporizing device.

The vaporizing device is provided with an identification code, by which the user terminal device can create a connection with the vaporizing device and create a communication thereto.

The cloud server is configured to store a program for identifying the identification code. Once the identification code is input, the administrator terminal device will be authorized to create a communication to the vaporizing device, such that various parameters is obtained and the control parameter is written.

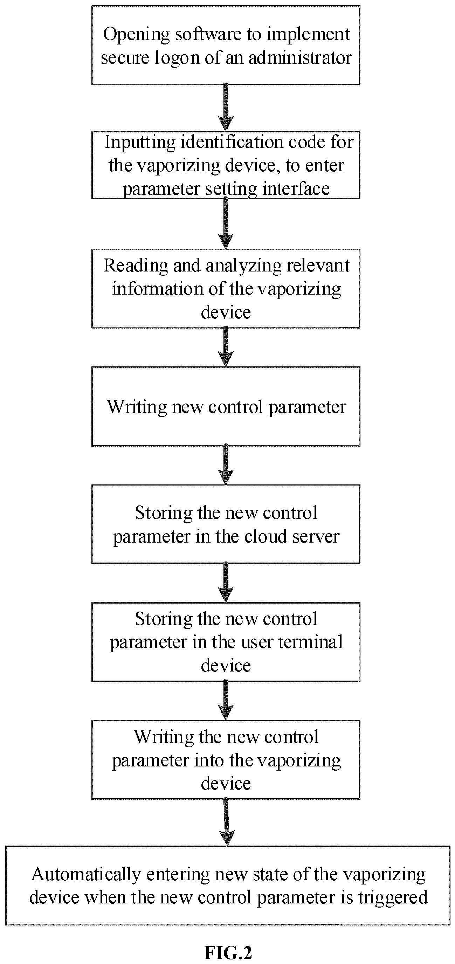

Referring to FIG.2, a method of controlling a vaporizing device according to the disclosure further comprises steps of:
(1) Opening software of the administrator terminal device, to implement secure logon of administrator;
(2) Inputting an identification code for the vaporizing device, to enter a parameter setting interface for the vaporizing device;
(3) Reading and analyzing relevant information of the vaporizing device;
(4) Writing a new control parameter based on a service condition of the vaporizing device and user's physical condition;
(5) Storing the new control parameter in the cloud server when the administrator terminal device is in communication with the cloud server;
(6) Storing the new control parameter in the user terminal device when the cloud server is in communication with the user terminal device;
(7) Writing the new control parameter into the vaporizing device when the user terminal device is in communication with the vaporizing device;
(8) Automatically entering a new state of the vaporizing device when the new control parameter is triggered.

In another embodiment on the basis of the preceding embodiment, the method further comprises the administrator terminal device and the vaporizing device being in direct communication with each other in a wired or wireless manner. Such method facilitates setting of new parameters on the spot by the administrator having face to face interview with the user.

The method according to the disclosure further comprises controlling the user's usage by means of the vaporizing device according to the new control parameter, and issuing an alarm by means of the administrator terminal device, or the user terminal device, or the vaporizing device when the new control parameter is triggered.

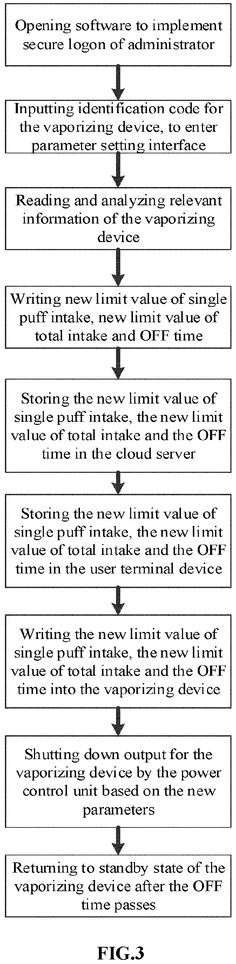

Referring to FIG.3, in terms of controlling an amount of aerosol intake, or an amount of medicine intake, or an amount of e-cigarette vapor intake, the method according to the disclosure further comprises steps of:
(1) Opening software of the administrator terminal device, to implement secure logon of administrator;
(2) Inputting an identification code for the vaporizing device, to enter a parameter management interface for the vaporizing device;
(3) Reading and analyzing relevant information of the vaporizing device;
(4) Writing a new limit value of single puff intake, a new limit value of total intake and an OFF time based on a service condition of the vaporizing device and user's personal information;
(5) Storing the new limit value of single puff intake, the new limit value of total intake and the OFF time in the cloud server when the administrator terminal device is in communication with the cloud server;
(6) Storing the new limit value of single puff intake, the new limit value of total intake and the OFF time in the user terminal device when the cloud server is in communication with the user terminal device;
(7) Writing the new limit value of single puff intake, the new limit value of total intake and the OFF time into the vaporizing device when the user terminal device is in communication with the vaporizing device;
(8) Implementing controlling of the vaporizing device based on the new limit value of single puff intake, the new limit value of total intake and the OFF time, and, when a value of single puff intake exceeds the limit value of single puff intake or a sum value of aerosol intake per unit time exceeds the limit value of total intake, shutting down output by means of the power control unit of the vaporizing device;
(9) Returning to a standby state of the vaporizing device after a period of the set OFF time passes since output is shut down by means of the power control unit of the vaporizing device.

In the above steps, the limit value of single puff intake refers to the limit value of the intake such as aerosol, or medicine, or e-cigarette vapor by each single puff of the user. The limit value of total intake refers to a limit value of the sum of aerosol intake per unit time, and the unit time is set to, for example, the past 24 hours. It is desired to set a limit value for the sum of aerosol intake of the user, to avoid overuse by the user. The OFF time refers to the period of time during which the vaporizing device is automatically shut down in the case that the intake of the user exceeds the limit value of single puff intake or the limit value of total intake, preventing the user from continually using the device in a short time to result in an overuse.

The method of controlling a vaporizing device according to the disclosure allows the authorized administrator (including regulatory authority, authorized medical personnel, and the like) to login and query the service condition of the vaporizing device by means of the administrator terminal device based on the identification code and to write a new control parameter when necessary. The method provides the vaporizing device with a targeted remote control in particular on the limit value of aerosol, medicine, or vapor intake, prevents the user from overusing the aerosol, medicine, or vapor in single puff or in a unit time, and avoids health hazards. According to the controlling method, the vaporizing device is operated in a condition that it is loaded with control programs. Thus, it is not necessary to remotely control the device on line all the time, and the vaporizing device can be normally operated even without network communication.

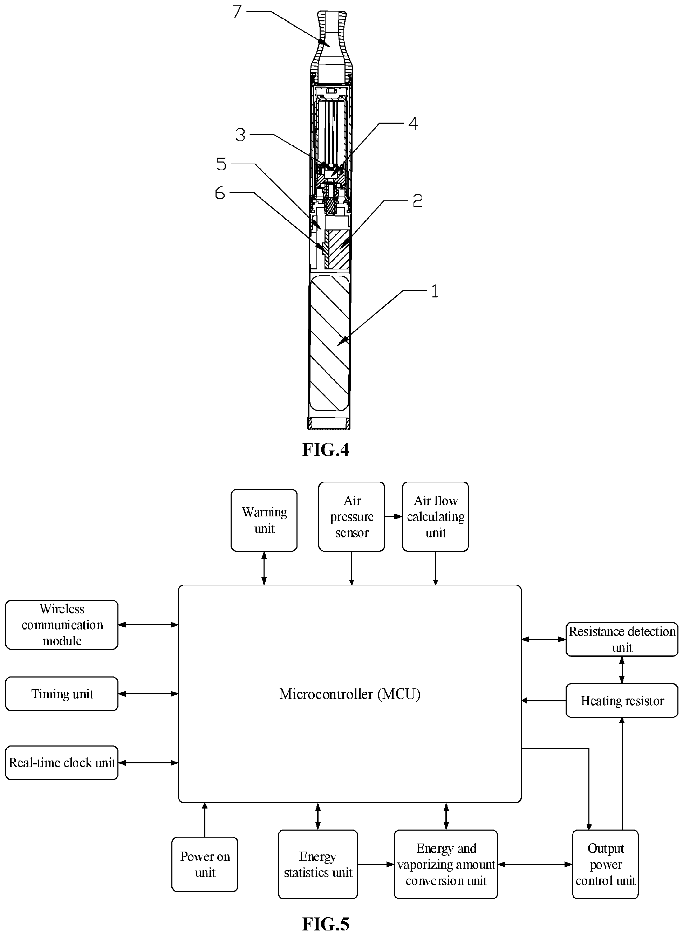

Referring to FIG.4, the vaporizing device of the disclosure comprises a battery 1, a control circuit board 2, a heating resistor 3, a vaporizing passage 4, and a mouth piece 7. The control circuit board 2 is arranged with a microcontroller MCU, a power control unit, and a communication unit, which are configured to execute instructions during operation, to implement the above controlling method. The microcontroller MCU is configured to compare the new control parameter with relevant parameters and then send corresponding control signal to the power control unit. On such basis, the power control unit is configured to control the output power to limit the user's usage.

Referring to FIG.4, in the vaporizing device of the disclosure which comprises a battery 1, a control circuit board 2, a heating resistor 3, a vaporizing passage 4, and a mouth piece 7, the heating resistor 3 is disposed in the vaporizing passage 4, and the battery 1 supplies power for the heating resistor 3. Under the control of control circuit board 2, the heating resistor 3 is energized to produce heat, and then vaporize liquid substances or paste-like substances, to generate aerosol or vapor for inhale. The user may inhale aerosol or vapor through the mouth piece 7. The vaporizing device of the disclosure further comprises a detecting air passage 5 and an air pressure sensor 6 disposed beside the detecting air passage 5 to detect the suction pressure.

Referring to FIG.5, the control circuit board 2 is arranged with the microcontroller MCU, an air flow calculating unit, an energy statistics unit, an energy and vaporizing amount conversion unit, and a power control unit, which are electrically connected with each other and configured to execute instructions during operation, to implement the above controlling method. The air flow calculating unit is configured to calculate the corresponding value of air flow based on the modeling volume of the detecting air passage and the change of suction pressure. The power control unit is configured to control output power for the heating resistor based on the value of air flow. The energy statistics unit is configured to calculate, based on the output power and the period of time, the energy output to the heating resistor. The energy and vaporizing amount conversion unit is configured to calculate the corresponding value of aerosol intake from the energy. The microcontroller MCU is configured to compare the value of aerosol intake with preset limit value of aerosol intake and then send corresponding control signal to the power control unit. The power control unit is configured to control the output power on such basis, to limit the aerosol intake.

Herein, the corresponding value of aerosol intake is calculated from the energy by means of the energy and vaporizing amount conversion unit, and then compared with the preset limit value of aerosol intake by means of the microcontroller MCU, to determine whether it is close to or exceeds the limit value. Then, the corresponding control signal is sent to the power control unit. After that, by means of the power control unit, the output power is reduced or shut down, thereby preventing the user from overusing the medicine, the vapor, or the like. In this way, an automatic control for the aerosol intake of the user is achieved.

The limit value of aerosol intake set in the microcontroller MCU comprises the limit value of single puff intake. When the value of single puff intake exceeds the limit value of single puff intake, the power control unit shuts down the output.

The limit value of aerosol intake set in the microcontroller MCU further comprises the limit value of total intake. When the sum value of aerosol intake per unit time exceeds the limit value of total intake, the power control unit shuts down the output.

Referring to FIG. 1, a control system for the vaporizing device according to the disclosure comprises the vaporizing device, the user terminal device, the cloud server, and the administrator terminal device which are in communication with each other. During communication, the administrator terminal device obtains various parameters of the vaporizing device, the administrator terminal device writes the new control parameter into the vaporizing device, the vaporizing device automatically enters a new state when the new control parameter is triggered. In this way, a control for the vaporizing device is achieved.

According to the disclosure, the administrator terminal device and the user terminal device include but are not limited to desktop computers, laptops, smart phones, I-PAD, and the like, which comprise an arithmetic and process unit and a communication unit and can implement the method of the disclosure during operation as long as being installed with specific programs or APPs. Usually, the administrator terminal device is used by the administrator, i.e., regulatory authority and authorized medical personnel. The user terminal device is matched with the vaporizing device and used by the user. The vaporizing device is activated only when the user logs in the user terminal device and the identification code is matched, thereby avoiding misuse by others or minors.

The vaporizing device of the disclosure limits the usage of the user according to the new control parameter. When the new control parameter is triggered, the alarm is issued by means of the administrator terminal device, or the user terminal device, or the vaporizing device. That is, these devices comprise an alarm unit. In addition, in the event of power on and off, stand-by time, the single puff intake exceeding the limit value, the sum intake exceeding the limit value, overheat, overload, or dry-heating, and the like, the alarm unit warns the user by an alarm in the form of a sound, a light, a vibration, and the like.

In another embodiment, the user terminal device is simplified and disposed in the vaporizing device, i.e., the user terminal device and the vaporizing device are incorporated, such that the vaporizing device has the function of the user terminal device, thereby facilitating usage and operation of the user.

## Claims

1. A method of controlling a vaporizing device, wherein the method comprises: by a communication between an administrator terminal device and a cloud server, a user terminal device and the vaporizing device, obtaining various parameters of the vaporizing device by means of the administrator terminal device, writing a new control parameter into the vaporizing device by means of the administrator terminal device, and, automatically entering a new state of the vaporizing device to achieve a control for the vaporizing device when the new control parameter is triggered;
wherein the method further comprises operating steps of:
(1) opening software of the administrator terminal device, to implement secure logon of an admini strator;
(2) inputting an identification code for the vaporizing device, to enter a parameter setting interface for the vaporizing device;
(3) reading and analyzing relevant information of the vaporizing device;
(4) writing a new control parameter based on a use condition of the vaporizing device and user's personal information;
(5) storing the new control parameter in the cloud server when the administrator terminal device is in communication with the cloud server;
(6) storing the new control parameter in the user terminal device when the cloud server is in communication with the user terminal device;
(7) writing the new control parameter into the vaporizing device when the user terminal device is in communication with the vaporizing device; wherein the new control parameter comprises a new limit value of aerosol intake; and
(8) automatically entering a new state of the vaporizing device when the new control parameter is triggered; wherein the automatically entering a new state of the vaporizing device when the new control parameter is triggered comprises: when a value of aerosol intake detected by means of the vaporizing device exceeds the new limit value of aerosol intake, shutting down power output by means of a power control unit of the vaporizing device for a period of set OFF time;
**characterized in that** the value of aerosol intake is detected by an air flow calculating unit, the power control unit, an energy statistics unit, and an energy and vaporizing amount conversion unit provided in the vaporizing device; wherein the air flow calculating unit is configured to calculate a value of air flow based on a modeling volume of a detecting air passage (5) and a change of suction pressure, the power control unit is configured to control output power that is output to a heating resistor (3) based on the value of air flow, the energy statistics unit is configured to calculate energy output to the heating resistor (3) based on the output power over a period of time, the energy and vaporizing amount conversion unit is configured to calculate the value of aerosol intake from the energy.

2. The method of controlling a vaporizing device according to claim 1, wherein the communication between an administrator terminal device and a cloud server, a user terminal device and a vaporizing device is implemented in such a manner that the administrator terminal device is in communication with the cloud server, the cloud server is in communication with the user terminal device, and the user terminal device is in communication with the vaporizing device.

3. The method of controlling a vaporizing device according to claim 1, wherein the method comprises the administrator terminal device and the vaporizing device being in direct communication with each other in a wired or wireless manner.

4. The method of controlling a vaporizing device according to claim 1, wherein the vaporizing device is configured with a first wired communication module or a first wireless communication module, wherein the first wired communication module or the first wireless communication module is in communication with a second wired communication module or a second wireless communication module of the user terminal device, to allow the user terminal device to read information of the vaporizing device and control the vaporizing device.

5. The method of controlling a vaporizing device according to claim 1, wherein the vaporizing device is provided with the identification code, by which the user terminal device is capable of creating a connection and communication with the vaporizing device.

6. The method of controlling a vaporizing device according to claim 5, wherein the cloud server is configured to store information or a program for identifying the identification code, the administrator terminal device is configured to be authorized by inputting the identification code, to create a communication with the vaporizing device, to obtain various parameters and write the control parameter.

7. The method of controlling a vaporizing device according to claim 1, wherein during the step of automatically entering a new state of the vaporizing device when the new control parameter is triggered, further issuing an alarm by means of the administrator terminal device, or the user terminal device, or the vaporizing device.

8. The method of controlling a vaporizing device according to claim 1, wherein the limit value of aerosol intake comprises a limit value of single puff intake, that is, when a value of single puff intake exceeds the limit value of single puff intake, shutting down output by means of the power control unit of the vaporizing device for the period of set OFF time, and/or the limit value of aerosol intake comprises a limit value of total intake, that is, when a sum value of aerosol intake per unit time exceeds the limit value of total intake, shutting down output by means of the power control unit of the vaporizing device for the period of set OFF time.

9. The method of controlling a vaporizing device according to claim 1, wherein the steps (4) to (8) specifically comprise operating steps of:
(1) writing a new limit value of single puff intake, a new limit value of total intake and an OFF time based on a service condition of the vaporizing device and user's personal information;
(2) storing the new limit value of single puff intake, the new limit value of total intake and the OFF time in the cloud server when the administrator terminal device is in communication with the cloud server;
(3) storing the new limit value of single puff intake, the new limit value of total intake and the OFF time in the user terminal device when the cloud server is in communication with the user terminal device;
(4) writing the new limit value of single puff intake, the new limit value of total intake and the OFF time into the vaporizing device when the user terminal device is in communication with the vaporizing device;
(5) implementing controlling of the vaporizing device based on the new limit value of single puff intake, the new limit value of total intake and the OFF time, and, when a value of single puff intake exceeds the limit value of single puff intake or a sum value of aerosol intake per unit time exceeds the limit value of total intake, shutting down output by means of the power control unit of the vaporizing device;
(6) returning to a standby state of the vaporizing device after the period of set OFF time passes since output is shut down by means of the power control unit of the vaporizing device.

10. A vaporizing device, comprising a battery (1), a control circuit board (2), a heating resistor (3), a vaporizing passage (4), a mouth piece (7), a detecting air passage (5), and an air pressure sensor (6) disposed in the detecting air passage (5) to detect suction pressure, wherein the control circuit board (2) is further arranged with a microcontroller (MCU), an air flow calculating unit, an energy statistics unit, an energy and vaporizing amount conversion unit, and a power control unit, which are configured to execute instructions during operation to implement the controlling method according to claim 1, the microcontroller (MCU) is configured to compare the corresponding value of aerosol intake with a preset limit value of aerosol intake and then send corresponding control signal to the power control unit, the power control unit is capable of shutting down power output for a certain period of time to limit aerosol intake.

11. A control system for a vaporizing device, wherein the control system comprises the vaporizing device according to claim 10, a user terminal device, a cloud server, and an administrator terminal device, which are in communication with each other, the administrator terminal device is configured to obtain various parameters of the vaporizing device during communication and write a new control parameter into the vaporizing device, and the vaporizing device is configured to automatically enter a new state to achieve a control for the vaporizing device when the new control parameter is triggered.

12. The control system for a vaporizing device according to claim 11, wherein the user terminal device is disposed in the vaporizing device.

## Patentansprüche

1. Verfahren zur Steuerung einer Verdampfungsvorrichtung, wobei das Verfahren umfasst: durch eine Kommunikation zwischen einem Administrator-Endgerät und einem Cloud-Server, einem Benutzer-Endgerät und der Verdampfungsvorrichtung, verschiedene Parameter der Verdampfungsvorrichtung durch das Administrator-Endgerät zu erhalten, einen neuen Steuerparameter durch das Administrator-Endgerät in die Verdampfungsvorrichtung zu schreiben und, automatisch in einen neuen Zustand der Verdampfungsvorrichtung einzutreten, um eine Steuerung für die Verdampfungsvorrichtung zu erreichen, wenn der neue Steuerparameter ausgelöst wird;
wobei das Verfahren ferner die folgenden Betriebsschritte umfasst:
(1) Öffnen der Software des Administrator-Endgeräts, um einen sicheren Login eines Administrators durchzuführen;
(2) Eingeben eines Identifikationscodes für die Verdampfungsvorrichtung, um eine Parameter-Einstelloberfläche für die Verdampfungsvorrichtung zu betreten;
(3) Lesen und Analysieren relevanter Informationen der Verdampfungsvorrichtung;
(4) Schreiben eines neuen Steuerparameters basierend auf einem Nutzungszustand der Verdampfungsvorrichtung und persönlichen Informationen des Benutzers;
(5) Speichern des neuen Steuerparameters im Cloud-Server, wenn das Administrator-Endgerät in Kommunikation mit dem Cloud-Server ist;
(6) Speichern des neuen Steuerparameters im Benutzer-Endgerät, wenn der Cloud-Server in Kommunikation mit dem Benutzer-Endgerät ist;
(7) Schreiben des neuen Steuerparameters in die Verdampfungsvorrichtung, wenn das Benutzer-Endgerät in Kommunikation mit der Verdampfungsvorrichtung ist; wobei der neue Steuerparameter einen neuen Grenzwert der Aerosolaufnahme umfasst; und
(8) automatisches Einleiten eines neuen Zustands der Verdampfungsvorrichtung, wenn der neue Steuerparameter ausgelöst wird; wobei das automatische Einleiten eines neuen Zustands der Verdampfungsvorrichtung, wenn der neue Steuerparameter ausgelöst wird, umfasst: wenn ein durch die Verdampfungsvorrichtung detektierter Wert der Aerosolaufnahme den neuen Grenzwert der Aerosolaufnahme überschreitet, Abschalten der Stromausgabe durch eine Leistungssteuereinheit der Verdampfungsvorrichtung für eine festgelegte AUS-Zeit;
**dadurch gekennzeichnet, dass** der Wert der Aerosolaufnahme durch eine Luftstromberechnungseinheit, die Leistungssteuereinheit, eine Energiestatistikeinheit und eine Energie- und Verdampfungsmengeumrechnungseinheit, die in der Verdampfungsvorrichtung vorgesehen sind, detektiert wird; wobei die Luftstromberechnungseinheit konfiguriert ist, einen Luftstromwert basierend auf einem Modellvolumen eines detektierenden Luftkanals (5) und einer Änderung des Saugdrucks zu berechnen, die Leistungssteuereinheit konfiguriert ist, die Ausgangsleistung, die an einen Heizwiderstand (3) ausgegeben wird, basierend auf dem Luftstromwert zu steuern, die Energiestatistikeinheit konfiguriert ist, die an den Heizwiderstand (3) ausgegebene Energie basierend auf der Ausgangsleistung über einen Zeitraum zu berechnen, und die Energie- und Verdampfungsmengeumrechnungseinheit konfiguriert ist, den Wert der Aerosolaufnahme aus der Energie zu berechnen.

2. Verfahren zur Steuerung einer Verdampfungsvorrichtung gemäß Anspruch 1, wobei die Kommunikation zwischen einem Administrator-Endgerät und einem Cloud-Server, einem Benutzer-Endgerät und einer Verdampfungsvorrichtung derart implementiert ist, dass das Administrator-Endgerät in Kommunikation mit dem Cloud-Server, der Cloud-Server in Kommunikation mit dem Benutzer-Endgerät und das Benutzer-Endgerät in Kommunikation mit der Verdampfungsvorrichtung ist.

3. Verfahren zur Steuerung einer Verdampfungsvorrichtung gemäß Anspruch 1, wobei das Verfahren umfasst, dass das Administrator-Endgerät und die Verdampfungsvorrichtung direkt miteinander in einer drahtgebundenen oder drahtlosen Weise kommunizieren.

4. Verfahren zur Steuerung einer Verdampfungsvorrichtung gemäß Anspruch 1, wobei die Verdampfungsvorrichtung mit einem ersten drahtgebundenen Kommunikationsmodul oder einem ersten drahtlosen Kommunikationsmodul konfiguriert ist, wobei das erste drahtgebundene Kommunikationsmodul oder das erste drahtlose Kommunikationsmodul in Kommunikation mit einem zweiten drahtgebundenen Kommunikationsmodul oder einem zweiten drahtlosen Kommunikationsmodul des Benutzer-Endgeräts steht, um dem Benutzer-Endgerät zu ermöglichen, Informationen der Verdampfungsvorrichtung zu lesen und die Verdampfungsvorrichtung zu steuern.

5. Verfahren zur Steuerung einer Verdampfungsvorrichtung gemäß Anspruch 1, wobei die Verdampfungsvorrichtung mit dem Identifikationscode versehen ist, durch den das Benutzer-Endgerät in der Lage ist, eine Verbindung und Kommunikation mit der Verdampfungsvorrichtung zu erstellen.

6. Verfahren zur Steuerung einer Verdampfungsvorrichtung gemäß Anspruch 5, wobei der Cloud-Server konfiguriert ist, Informationen oder ein Programm zur Identifizierung des Identifikationscodes zu speichern, das Administrator-Endgerät konfiguriert ist, durch Eingabe des Identifikationscodes autorisiert zu werden, eine Kommunikation mit der Verdampfungsvorrichtung herzustellen, um verschiedene Parameter zu erhalten und den Steuerparameter zu schreiben.

7. Verfahren zur Steuerung einer Verdampfungsvorrichtung gemäß Anspruch 1, wobei während des Schritts des automatischen Einleitens eines neuen Zustands der Verdampfungsvorrichtung, wenn der neue Steuerparameter ausgelöst wird, ferner ein Alarm durch das Administrator-Endgerät oder das Benutzer-Endgerät oder die Verdampfungsvorrichtung ausgegeben wird.

8. Verfahren zur Steuerung einer Verdampfungsvorrichtung gemäß Anspruch 1, wobei der Grenzwert der Aerosolaufnahme einen Grenzwert der Einzelzugaufnahme umfasst, das heißt, wenn ein Wert der Einzelzugaufnahme den Grenzwert der Einzelzugaufnahme überschreitet, Abschalten der Ausgabe durch die Leistungssteuereinheit der Verdampfungsvorrichtung für die festgelegte AUS-Zeit, und/oder der Grenzwert der Aerosolaufnahme umfasst einen Grenzwert der Gesamtaufnahme, das heißt, wenn ein Summenwert der Aerosolaufnahme pro Zeiteinheit den Grenzwert der Gesamtaufnahme überschreitet, Abschalten der Ausgabe durch die Leistungssteuereinheit der Verdampfungsvorrichtung für die festgelegte AUS-Zeit.

9. Verfahren zur Steuerung einer Verdampfungsvorrichtung gemäß Anspruch 1, wobei die Schritte (4) bis (8) spezifisch die folgenden Betriebsschritte umfassen:
(1) Schreiben eines neuen Grenzwerts der Einzelzugaufnahme, eines neuen Grenzwerts der Gesamtaufnahme und einer AUS-Zeit basierend auf einem Servicezustand der Verdampfungsvorrichtung und den persönlichen Informationen des Benutzers;
(2) Speichern des neuen Grenzwerts der Einzelzugaufnahme, des neuen Grenzwerts der Gesamtaufnahme und der AUS-Zeit im Cloud-Server, wenn das Administrator-Endgerät in Kommunikation mit dem Cloud-Server ist;
(3) Speichern des neuen Grenzwerts der Einzelzugaufnahme, des neuen Grenzwerts der Gesamtaufnahme und der AUS-Zeit im Benutzer-Endgerät, wenn der Cloud-Server in Kommunikation mit dem Benutzer-Endgerät ist;
(4) Schreiben des neuen Grenzwerts der Einzelzugaufnahme, des neuen Grenzwerts der Gesamtaufnahme und der AUS-Zeit in die Verdampfungsvorrichtung, wenn das Benutzer-Endgerät in Kommunikation mit der Verdampfungsvorrichtung ist;
(5) Implementieren der Steuerung der Verdampfungsvorrichtung basierend auf dem neuen Grenzwert der Einzelzugaufnahme, dem neuen Grenzwert der Gesamtaufnahme und der AUS-Zeit, und, wenn ein Wert der Einzelzugaufnahme den Grenzwert der Einzelzugaufnahme oder ein Summenwert der Aerosolaufnahme pro Zeiteinheit den Grenzwert der Gesamtaufnahme überschreitet, Abschalten der Ausgabe durch die Leistungssteuereinheit der Verdampfungsvorrichtung;
(6) Rückkehr in einen Standby-Zustand der Verdampfungsvorrichtung nach Ablauf der festgelegten AUS-Zeit, seit die Ausgabe durch die Leistungssteuereinheit der Verdampfungsvorrichtung abgeschaltet wurde.

10. Eine Verdampfungsvorrichtung, umfassend eine Batterie (1), eine Steuerplatine (2), einen Heizwiderstand (3), einen Verdampfungskanal (4), ein Mundstück (7), einen detektierenden Luftkanal (5) und einen Luftdrucksensor (6), der im detektierenden Luftkanal (5) angeordnet ist, um den Saugdruck zu detektieren, wobei die Steuerplatine (2) ferner mit einem Mikrocontroller (MCU), einer Luftstromberechnungseinheit, einer Energiestatistikeinheit, einer Energie- und Verdampfungsmengeumrechnungseinheit und einer Leistungssteuereinheit ausgestattet ist, die konfiguriert sind, Anweisungen während des Betriebs auszuführen, um das Steuerungsverfahren gemäß Anspruch 1 zu implementieren, wobei der Mikrocontroller (MCU) konfiguriert ist, den entsprechenden Wert der Aerosolaufnahme mit einem voreingestellten Grenzwert der Aerosolaufnahme zu vergleichen und dann ein entsprechendes Steuersignal an die Leistungssteuereinheit zu senden, die Leistungssteuereinheit in der Lage ist, die Stromausgabe für eine bestimmte Zeit zu stoppen, um die Aerosolaufnahme zu begrenzen.

11. Ein Steuerungssystem für eine Verdampfungsvorrichtung, wobei das Steuerungssystem die Verdampfungsvorrichtung gemäß Anspruch 10, ein Benutzer-Endgerät, einen Cloud-Server und ein Administrator-Endgerät umfasst, die miteinander in Kommunikation stehen, wobei das Administrator-Endgerät konfiguriert ist, verschiedene Parameter der Verdampfungsvorrichtung während der Kommunikation zu erhalten und einen neuen Steuerparameter in die Verdampfungsvorrichtung zu schreiben, und die Verdampfungsvorrichtung konfiguriert ist, automatisch in einen neuen Zustand zu treten, um eine Steuerung für die Verdampfungsvorrichtung zu erreichen, wenn der neue Steuerparameter ausgelöst wird.

12. Das Steuerungssystem für eine Verdampfungsvorrichtung gemäß Anspruch 11, wobei das Benutzer-Endgerät in der Verdampfungsvorrichtung angeordnet ist.

## Revendications

1. Procédé de contrôle d'un dispositif de vaporisation, le procédé comprenant : par une communication entre un terminal administrateur et un serveur cloud, un terminal utilisateur et le dispositif de vaporisation, obtenir divers paramètres du dispositif de vaporisation au moyen du terminal administrateur, écrire un nouveau paramètre de contrôle dans le dispositif de vaporisation au moyen du terminal administrateur, et, entrer automatiquement dans un nouvel état du dispositif de vaporisation pour réaliser un contrôle pour le dispositif de vaporisation lorsque le nouveau paramètre de contrôle est déclenché;
le procédé comprenant en outre les étapes suivantes :
(1) ouvrir le logiciel du terminal administrateur pour effectuer une connexion sécurisée d'un administrateur ;
(2) saisir un code d'identification pour le dispositif de vaporisation pour entrer dans une interface de réglage des paramètres pour le dispositif de vaporisation ;
(3) lire et analyser les informations pertinentes du dispositif de vaporisation ;
(4) écrire un nouveau paramètre de contrôle basé sur un état d'utilisation du dispositif de vaporisation et les informations personnelles de l'utilisateur ;
(5) stocker le nouveau paramètre de contrôle dans le serveur cloud lorsque le terminal administrateur est en communication avec le serveur cloud ;
(6) stocker le nouveau paramètre de contrôle dans le terminal utilisateur lorsque le serveur cloud est en communication avec le terminal utilisateur ;
(7) écrire le nouveau paramètre de contrôle dans le dispositif de vaporisation lorsque le terminal utilisateur est en communication avec le dispositif de vaporisation ; le nouveau paramètre de contrôle comprenant une nouvelle valeur limite de l'apport d'aérosol ; et
(8) entrer automatiquement dans un nouvel état du dispositif de vaporisation lorsque le nouveau paramètre de contrôle est déclenché ; l'entrée automatique dans un nouvel état du dispositif de vaporisation lorsque le nouveau paramètre de contrôle est déclenché comprenant : lorsque la valeur de l'apport d'aérosol détectée par le dispositif de vaporisation dépasse la nouvelle valeur limite de l'apport d'aérosol, arrêter la sortie de puissance au moyen d'une unité de contrôle de puissance du dispositif de vaporisation pour une période de temps défini en mode OFF ;
**caractérisé en ce que** la valeur de l'apport d'aérosol est détectée par une unité de calcul du flux d'air, l'unité de contrôle de puissance, une unité de statistiques énergétiques et une unité de conversion de la quantité d'énergie et de vaporisation prévues dans le dispositif de vaporisation ; l'unité de calcul du flux d'air étant configurée pour calculer une valeur du flux d'air basée sur un volume modélisé d'un passage d'air de détection (5) et une variation de la pression de succion, l'unité de contrôle de puissance étant configurée pour contrôler la puissance de sortie délivrée à une résistance chauffante (3) en fonction de la valeur du flux d'air, l'unité de statistiques énergétiques étant configurée pour calculer l'énergie délivrée à la résistance chauffante (3) en fonction de la puissance de sortie sur une période de temps, et l'unité de conversion de la quantité d'énergie et de vaporisation étant configurée pour calculer la valeur de l'apport d'aérosol à partir de l'énergie.

2. Procédé de contrôle d'un dispositif de vaporisation selon la revendication 1, la communication entre un terminal administrateur et un serveur cloud, un terminal utilisateur et un dispositif de vaporisation étant mise en oeuvre de telle manière que le terminal administrateur est en communication avec le serveur cloud, le serveur cloud est en communication avec le terminal utilisateur, et le terminal utilisateur est en communication avec le dispositif de vaporisation.

3. Procédé de contrôle d'un dispositif de vaporisation selon la revendication 1, le procédé comprenant que le terminal administrateur et le dispositif de vaporisation communiquent directement entre eux de manière filaire ou sans fil.

4. Procédé de contrôle d'un dispositif de vaporisation selon la revendication 1, le dispositif de vaporisation étant configuré avec un premier module de communication filaire ou un premier module de communication sans fil, le premier module de communication filaire ou le premier module de communication sans fil étant en communication avec un second module de communication filaire ou un second module de communication sans fil du terminal utilisateur pour permettre au terminal utilisateur de lire les informations du dispositif de vaporisation et de contrôler le dispositif de vaporisation.

5. Procédé de contrôle d'un dispositif de vaporisation selon la revendication 1, le dispositif de vaporisation étant pourvu du code d'identification par lequel le terminal utilisateur est capable de créer une connexion et une communication avec le dispositif de vaporisation.

6. Procédé de contrôle d'un dispositif de vaporisation selon la revendication 5, le serveur cloud étant configuré pour stocker des informations ou un programme pour identifier le code d'identification, le terminal administrateur étant configuré pour être autorisé par la saisie du code d'identification pour créer une communication avec le dispositif de vaporisation, obtenir divers paramètres et écrire le paramètre de contrôle.

7. Procédé de contrôle d'un dispositif de vaporisation selon la revendication 1, lors de l'étape d'entrée automatique dans un nouvel état du dispositif de vaporisation lorsque le nouveau paramètre de contrôle est déclenché, émettre en outre une alarme par le biais du terminal administrateur, ou du terminal utilisateur, ou du dispositif de vaporisation.

8. Procédé de contrôle d'un dispositif de vaporisation selon la revendication 1, la valeur limite de l'apport d'aérosol comprenant une valeur limite de l'apport par bouffée unique, c'est-à-dire, lorsqu'une valeur de l'apport par bouffée unique dépasse la valeur limite de l'apport par bouffée unique, arrêter la sortie au moyen de l'unité de contrôle de puissance du dispositif de vaporisation pour la période de temps définie en mode OFF, et/ou la valeur limite de l'apport d'aérosol comprenant une valeur limite de l'apport total, c'est-à-dire, lorsqu'une valeur sommaire de l'apport d'aérosol par unité de temps dépasse la valeur limite de l'apport total, arrêter la sortie au moyen de l'unité de contrôle de puissance du dispositif de vaporisation pour la période de temps définie en mode OFF.

9. Procédé de contrôle d'un dispositif de vaporisation selon la revendication 1, les étapes (4) à (8) comprenant spécifiquement les étapes suivantes :
(1) écrire une nouvelle valeur limite de l'apport par bouffée unique, une nouvelle valeur limite de l'apport total et un temps OFF basé sur un état de service du dispositif de vaporisation et les informations personnelles de l'utilisateur ;
(2) stocker la nouvelle valeur limite de l'apport par bouffée unique, la nouvelle valeur limite de l'apport total et le temps OFF dans le serveur cloud lorsque le terminal administrateur est en communication avec le serveur cloud ;
(3) stocker la nouvelle valeur limite de l'apport par bouffée unique, la nouvelle valeur limite de l'apport total et le temps OFF dans le terminal utilisateur lorsque le serveur cloud est en communication avec le terminal utilisateur ;
(4) écrire la nouvelle valeur limite de l'apport par bouffée unique, la nouvelle valeur limite de l'apport total et le temps OFF dans le dispositif de vaporisation lorsque le terminal utilisateur est en communication avec le dispositif de vaporisation ;
(5) mettre en oeuvre le contrôle du dispositif de vaporisation basé sur la nouvelle valeur limite de l'apport par bouffée unique, la nouvelle valeur limite de l'apport total et le temps OFF, et, lorsqu'une valeur de l'apport par bouffée unique dépasse la valeur limite de l'apport par bouffée unique ou qu'une valeur sommaire de l'apport d'aérosol par unité de temps dépasse la valeur limite de l'apport total, arrêter la sortie au moyen de l'unité de contrôle de puissance du dispositif de vaporisation ;
(6) revenir à un état de veille du dispositif de vaporisation après que la période de temps définie en mode OFF soit écoulée depuis l'arrêt de la sortie au moyen de l'unité de contrôle de puissance du dispositif de vaporisation.

10. Un dispositif de vaporisation comprenant une batterie (1), une carte de circuit de contrôle (2), une résistance chauffante (3), un passage de vaporisation (4), un embout buccal (7), un passage d'air de détection (5), et un capteur de pression d'air (6) disposé dans le passage d'air de détection (5) pour détecter la pression de succion, la carte de circuit de contrôle (2) étant en outre agencée avec un microcontrôleur (MCU), une unité de calcul du flux d'air, une unité de statistiques énergétiques, une unité de conversion de la quantité d'énergie et de vaporisation, et une unité de contrôle de puissance, qui sont configurés pour exécuter des instructions pendant le fonctionnement pour mettre en oeuvre le procédé de contrôle selon la revendication 1, le microcontrôleur (MCU) étant configuré pour comparer la valeur correspondante de l'apport d'aérosol avec une valeur limite prédéfinie de l'apport d'aérosol, puis envoyer un signal de contrôle correspondant à l'unité de contrôle de puissance, l'unité de contrôle de puissance étant capable d'arrêter la sortie de puissance pour une certaine période de temps afin de limiter l'apport d'aérosol.

11. Un système de contrôle pour un dispositif de vaporisation, le système de contrôle comprenant le dispositif de vaporisation selon la revendication 10, un terminal utilisateur, un serveur cloud et un terminal administrateur, qui sont en communication les uns avec les autres, le terminal administrateur étant configuré pour obtenir divers paramètres du dispositif de vaporisation pendant la communication et écrire un nouveau paramètre de contrôle dans le dispositif de vaporisation, et le dispositif de vaporisation étant configuré pour entrer automatiquement dans un nouvel état afin de réaliser un contrôle pour le dispositif de vaporisation lorsque le nouveau paramètre de contrôle est déclenché.

12. Le système de contrôle pour un dispositif de vaporisation selon la revendication 11, le terminal utilisateur étant disposé dans le dispositif de vaporisation.
